# EUROPEAN PATENT APPLICATION

(11) **EP 1 547 553 A2**
(43) Date of publication of application: **29.06.2005**
(21) Application number: 04380247.9
(22) Date of filing: 02.12.2004
(51) Int. Cl.: A61F 13/15

(54) **Method for applying elastic adhesive**

(30) Priority: 19.12.2003 MX pa04000089
(71) Applicant: Grupo P.I. Mabe, S.A. de C.V., 72230 Puebla, Estado de Puebla (MX)
(72) Inventor: Corona Carlos, Alberto, Col. La Loma 72230 Puebla (MX); Canales Espinosa de Los Monteros, Carlos, Col. La Loma 72230 Puebla (MX); Sanchez Fernandez, Lucia, Col. La Loma 72230 Puebla (MX)
(74) Representative: Gil Vega, Victor

(57) **Abstract**

A method of applying an elastic adhesive, which is applied while hot, such that same contracts upon cooling is disclosed, the adhesive being applied in a zig-zag pattern over the material or between the materials to be elasticized, such that even though the application is made in the longitudinal direction, transverse elasticizing of the materials is achieved.

## Description

### Background of the Invention

Generally, all disposable absorbent articles, such as disposable diapers for babies or for adults, disposable training pants, sanitary napkins or disposable articles for incontinence, posses one or more elastic zones to provide a better fit to the body of the user thereof, as well as to make them more comfortable and safer. By way of example, the invention will be described in regard to a disposable diaper, except for the fact that it is applicable to any of the previously mentioned articles.

A disposable diaper is basically comprised of a hydrophobic outer sheet, a hydrophillic inner sheet and an absorbent core disposed between both sheets, as well as a fastening system; the inner and outer sheets are longer and wider than the absorbent core and provide the diaper with an anatomic shape; the parts that protrude from the absorbent core constitute the periphery of the article. The diaper has one or more elastic zones on those areas that will be disposed in the legs and the waist of the user. Generally, in order to achieve elasticity in said zones, elastic materials that are disposed stressed between the outer and inner sheets are used, and they are joined to each other by any known means such as a hot-melt adhesive, ultra-sound, etc. It is also desirable that some of the zones in the periphery are elastic in order to provide the user with a better fit. Elastic materials have begun to be used for the upper and/or outer sheets in order to achieve this; however, use of same significantly increases the costs of the article. Another manner of achieving elasticity is the use of an adhesive with elastic properties between the inner and outer sheets in those zones that require same, the application of adhesives being in any shapes and in different directions.

There are many proposals regarding methods for applying an adhesive; as an example, US 4,687,137, assigned to Nordson Corporation, discloses an adhesive supplying apparatus having a nozzle by means of which adhesive rings are applied, some rings being continuous and some other rings being intermittent; US 6,651,906, also assigned to Nordson Corporation, relates to spiral application of adhesive; US 5,056,571, assigned to H.B. Fuller Licensing & Financing, discloses an adhesive that can be applied in the manner of multiple lines or by means of spray; US 5,421,941, assigned to J&M Laboratories, Inc., relates to a method of spray application of adhesive by means of air, so to enlarge the application area.

On the other hand, inasmuch as elastic adhesives are concerned, US 4,259,220, assigned to H.B. Fuller Company, discloses a type of elastic adhesive by means of which elongations of up to about 500% are achieved; the adhesive needs to be pre-extruded in order for it to be applied later on the area to be elasticized; US 4,761,198, assigned to National Starch and Chemical Corporation, like the preceding patent, describes an elastic adhesive formulation which, after forming self-adhering elastic bands, can be used instead of the elastic bands which are common in the waist and crotch zones of a disposable diaper; US 2003011166 A1, Duane et al., disclosed a method to elasticize the waist zone and the crotch zone by employing an elastic adhesive on a non-woven fabric, such that the adhesive is applied over the film in order to thereafter form the absorbent article with the film already contracted. None of the above-identified patent documents describe a type of elastic adhesive to be applied while hot directly over the material or between the materials to be elasticized, so that it contracts upon cooling, thus elasticizing the application area in the sense of same, which generally is done in the machine direction, achieving elongation of the article in this direction; however, what is desirable, particularly in the waist zone and in the periphery of the disposable absorbent articles is a transversely elastic article, which is difficult to achieve using an elastic adhesive.

Therefore, the present invention proposes a method of applying an elastic adhesive which is applied while hot directly on the material(s) to be elasticized and which contracts upon cooling, so that even though the application is made in the longitudinal direction, transverse elongation of the material(s) is achieved; for this purpose, the application of an elastic adhesive between the upper and lower sheets of a disposable absorbent article is described, in the form of a zig-zag pattern, and preferable in the form of parallel applications, that is two or more zig-zag pattern applications of an adhesive in a parallel manner. The desired transverse elastic effect is thus achieved.

### Objects of the Invention

An object of the present invention consists of the use of an elastic adhesive applied while hot so that same is applied directly over or between the materials to elasticize, such that same contracts upon cooling.

A further object of the invention consists of the longitudinal application of adhesive such that transverse elongation of the material(s) is achieved.

An additional object of the invention is to achieve a zig-zag pattern application of elastic adhesive such that the materials are able to transversely elongate.

Another object of the invention is to carry out several parallel, zig-zag pattern applications to each other in order to achieve an elasticizing zone.

Another object of the invention in to employ the elastic adhesive application method to elasticize certain zones in a disposable absorbent article.

### Brief Description of the Drawings

Figure 1 shows the application of elastic adhesive in a zig-zag pattern of the present invention.
Figure 2 illustrates a series of parallel applications of elastic adhesive in a zig-zag pattern.
Figure 3 shows a disposable diaper with transverse elasticity zones achieved by means of the application of elastic adhesive in a zig-zag pattern.

### Detailed Description of the Invention

The present invention relates to a method of applying an elastic adhesive such that, even though the application may be done in the longitudinal direction, transverse elasticity is achieved in the material(s) over or between which it is applied; specifically, the invention relates to a method of applying an elastic adhesive which is applied while hot such that it contracts upon cooling, as well as its use in the manufacture of disposable absorbent articles such as disposable diapers, training pants, sanitary napkins and disposable articles for incontinence, such that the article is elasticized in specific zones thereof.

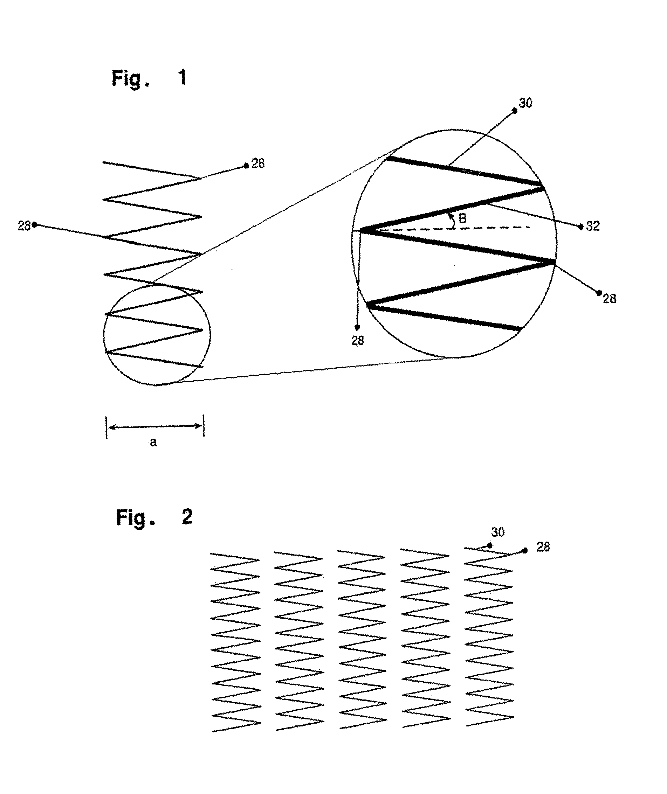

The method consists of applying the elastic adhesive in a zig-zag pattern by means of an application nozzle which constantly moves from left to right, resulting in an application such as that shown in Figure 1, with the peaks (28) of the zig-zag pattern about the left and right parts of the application, each zig-zag pattern being formed by a first line (30) which runs to the right, by a peak (28), and by a second line (32) which runs to the left, such that the latter will join with another line (30) to form a new peak (28) and so on. Each of the lines running to the right or to the left is applied at a maximum angle "B" in regard to a horizontal line of about 35 degrees. The application in a zig-zag pattern is made at such a speed that a minimum application of about five peaks (28) per centimeter is achieved, the maximum amplitude "a" of each application being of about 30 mm.

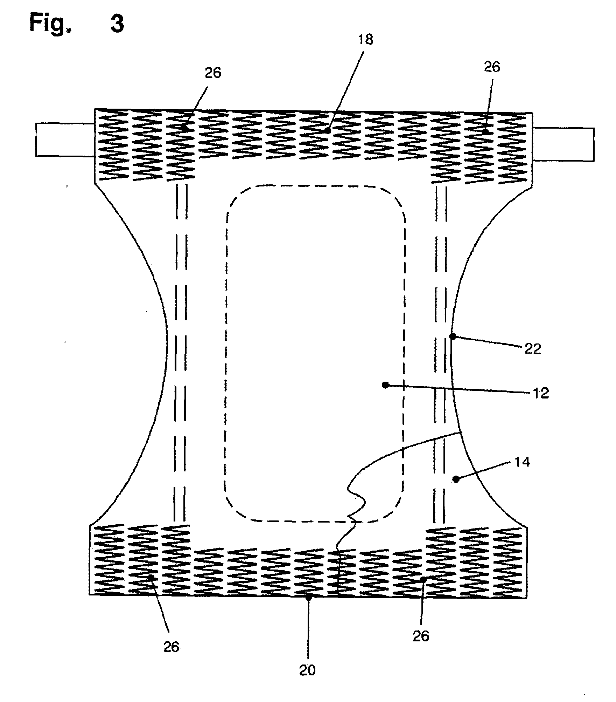

In this manner, the materials between which the elastic adhesive is applied in a zig-zag pattern are contracted at the application angle, in the zone of application. Depending on the area to be elasticized, the necessary applications will be carried out in a parallel manner, such as shown in Figure 2, achieving an integral effect for all applications, such that the article contracts transversely. Thus, the zone(s) of the article can be elasticized in those areas which require elasticizing by making parallel, zig-zag pattern applications of elastic adhesive.

By way of example, Figure 3 shown a disposable diaper with application of elastic adhesive in a zig-zag pattern between the upper sheet (12) and the lower sheet (14) in the waist areas (18,20) and the ears (26) of same. These zones of the article are elasticized without the need of using any other type of higher-cost materials.

While the invention has been described as a function of a currently preferred embodiment, it is apparent that changes and variations thereof can be made, all such changes and variations falling with the scope and spirt of the invention, as defined in the appended claims.

## Claims

1. A hot-applied elastic adhesive, **characterized in that** it is applied directly over or between the materials to be elasticized, such that it contracts upon cooling, achieving the elasticizing of the material(s).

2. A method of applying an elastic adhesive such as that recited in claim 1, **characterized in that** the adhesive is applied in a zig-zag pattern, with lines running to the right and lines running to the left, such that they form the peaks of the zig-zag pattern when they join each other.

3. A method of applying an elastic adhesive, as recited in claim 2, **characterized in that** the application in a zig-zag pattern is made at a speed such that a minimum application of about five peaks per centimeter is achieved.

4. A method of applying an elastic adhesive, as recited in claim 2, **characterized in that** the angle of application of the lines of the zig-zag pattern has a maximum value of about 35 degrees with respect to a horizontal line.

5. A method of applying an elastic adhesive, as recited in claim 2, **characterized in that** the maximum amplitude of each application in a zig-zag pattern is of about 30 mm.

6. A method of applying an elastic adhesive, as recited in claim 2, **characterized in that** several parallel applications in a zig-zag pattern are made to elasticize a particular zone.

7. A disposable absorbent article, basically comprising an inner sheet, an outer sheet and an absorbent core disposed between both sheets, such that the inner and outer sheets are longer and wider than the absorbent core, forming the periphery of the article, **characterized in that** an elastic adhesive is used, the elastic adhesive being applied in a zig-zag pattern between the inner and outer sheets, wherein said adhesive is the adhesive of claim 1, which is applied in accordance with the method of claim 2, to elasticize zones of the article.
